# EUROPEAN PATENT SPECIFICATION

(11) **EP 2 687 250 B1**
(45) Date of publication and mention of the grant of the patent: **20.02.2019**
(21) Application number: 13163509.6
(22) Date of filing: 12.04.2013
(51) Int. Cl.: A61M 3/02, A61M 25/00, A61M 31/00

(54) **Nasal flushing device**
Nasenspülvorrichtung
Dispositif de rinçage nasal

(30) Priority: 18.07.2012 TW 101125880
(43) Date of publication of application: 22.01.2014
(73) Proprietor: Wu, Lih-Chiu, 80045 Kaohsiung City (TW); Lee, Hui-Shuan, 80045 Kaohsiung City (TW)
(72) Inventor: WU, Lih-Chiu, 80045 Kachsiung City (TW); LEE, Hui-Shuan, 80045 Kachsiung City (TW); LEE, Tze-Yu, 80045 Kaohsiung City (TW)
(74) Representative: Lang, Christian

(56) References cited:
- WO-A1-2013/142333
- WO-A2-2008/091652
- CN-Y- 201 346 296
- RU-C2- 2 252 040
- US-A- 6 123 697
- US-A1- 2004 039 352
- US-A1- 2011 297 240
- US-B2- 7 959 623

## Description

### BACKGROUND OF THE INVENTION

### 1. Field of the Invention

The present invention relates to a nasal flushing device, and more particularly to a portable nasal flushing device.

### 2. Description of the Related Art

Recently, with climate change and over development, air pollution becomes a serious issue in our daily life. According to Medscape website (http://emedicine.medscape.com/article/232791-overview#a0156) in USA, every 1000 people, there are 146 people have chronic nasosinusitis, and roughly estimation there are about 18 to 22 million doctor visits and 3.4 to 5.0 billion direct medical spent. European region, not found clinics data, but based on the Allergy journal (Allergy volume 66, Issue 9, page 1216-1223 September 9 2011) pointed out that the European region also prevalence of up to 10.9%. So far, according to Medscape and Mayo Clinic websites, there is no cure for chronic nasosinusitis. The main symptoms are nasal obstruction, post nasal drip and cough. Surgical solution is only suitable for some cases, and antibiotics are effective for acute bacterial infection rather than chronic conditions. At home, nasal irrigation is recommended as a long term treatment of the symptoms.

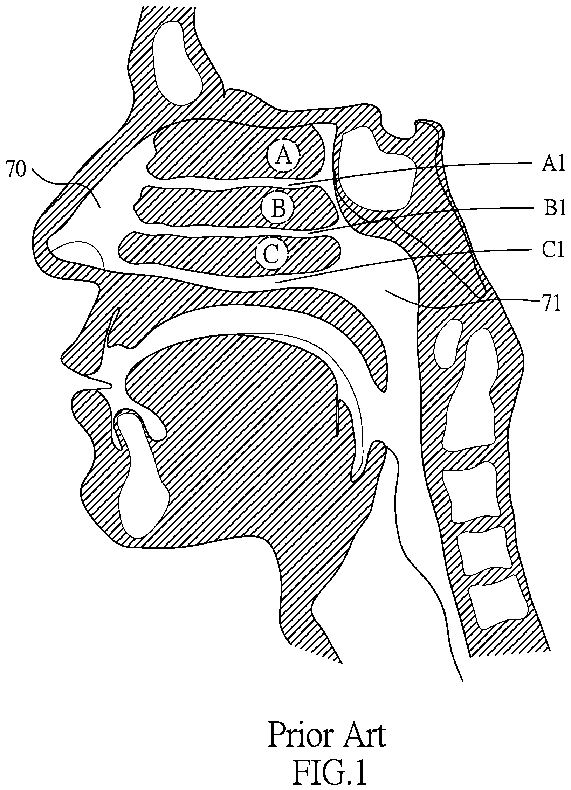

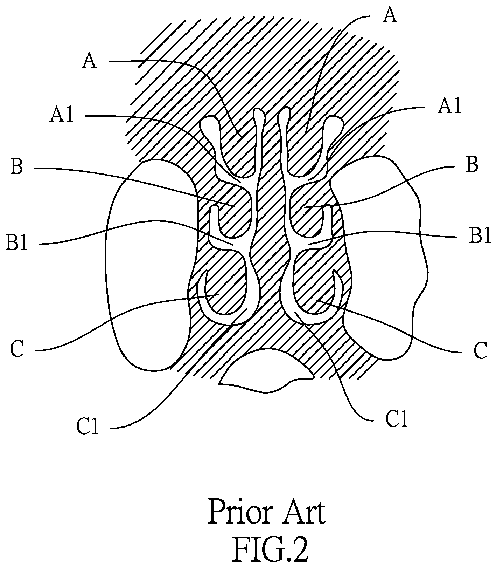

Please refer to FIG. 1. In the nasal cavity between the nasal vestibule and nasopharynx is divided by the superior turbinate A, the middle turbinate B, the inferior turbinate C, the upper nasal meatus A1, the middle nasal meatus B1 and the lower nasal meatus C1. All secretion from the paranasal sinuses is exited from the several openings located in the upper nasal meatus A1 and the middle nasal meatus. Therefore, thick tenacious secretion generated by chronic nasosinusitis mainly builds up the upper nasal meatus and the middle nasal meatus nasal congestion, which causes nasal obstruction.

In order to moisturize the nasal cavity, the options are warm damp facial cover, warm steam inhalation and nasal irrigations such as neti pot can be applied. However, the first two solutions cannot bring the moisture directly into the nasal cavity.

Furthermore, most of improvements for the nasal irrigation devices in the market are related to supplying mechanisms instead of changing water flow direction.

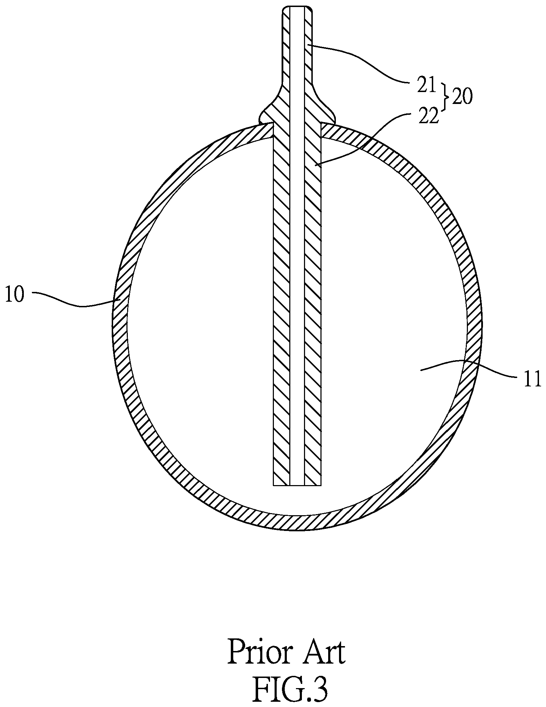

Please refer to FIG. 3.

As Taiwan patent No. M418689 discloses a nasal irrigation device has: an applying portion 10 and a nozzle 20. The applying portion 10 has an inner space 11 and the nozzle 20 has an opening 21 and an extending tube 22 reaching towards to the inner space 11 of the applying portion 10. When the inner space 11 is filled with water and compressed to push the water into the extending tube 22 and exit from the opening 21.

As previous description, nasosinusitis patient needs to moist the upper nasal meatus A1 and the middle nasal meatus B1, the above mentioned nasal irrigation device can bring water to the nasal vestibule. However, due to the dimension of the nozzle 20, it can only provide a single direction spout at the nasal vestibule, which can just reach to the lower nasal meatus not deep into the upper nasal meatus A1 and the middle nasal meatus B1 to treat the symptoms correctly.

Moreover, the spout provided by the device might be too strong for nasal and nasopharyngeal mucosa or choke the patient.

Therefore, it is desirable to provide a nasal flushing device to mitigate and/or obviate the aforementioned problems.

CN 201 346 296 Y, US 6 123 697 A, US 2011/297240 A1, US 2004/039352 A1, WO 2008/091652 A2, RU 2 252 040 C2, WO 2013/142333 A1 disclose relevant prior art.

### SUMMARY OF THE INVENTION

The invention is defined in the appended claims.

An objective of the present invention is to provide an easy nasal flushing device.

In order to achieve the above mentioned objective, the nasal flushing device includes a catheter unit and an injection unit.

The catheter unit has a catheter body and a plurality of apertures formed on the catheter body, the catheter body having an open end and a closed end opposite the open end. The injection unit has a detachable syringe and a putter set provided in the syringe.

The nasal flushing device utilizes the catheter unit to deeply rinse the nasal cavity, and the combination of the catheter unit, the connecting unit, and the injection unit provides portability to the user.

Other objects, advantages, and novel features of the invention will become more apparent from the following detailed description when taken in conjunction with the accompanying drawings.

### BRIEF DESCRIPTION OF THE DRAWINGS

FIG. 1 is anatomy cross-sectional view of the nasal cavity.
FIG. 3 is a cross-sectional view of a nasal irrigation device disclosed in Taiwan patent No. M418689.

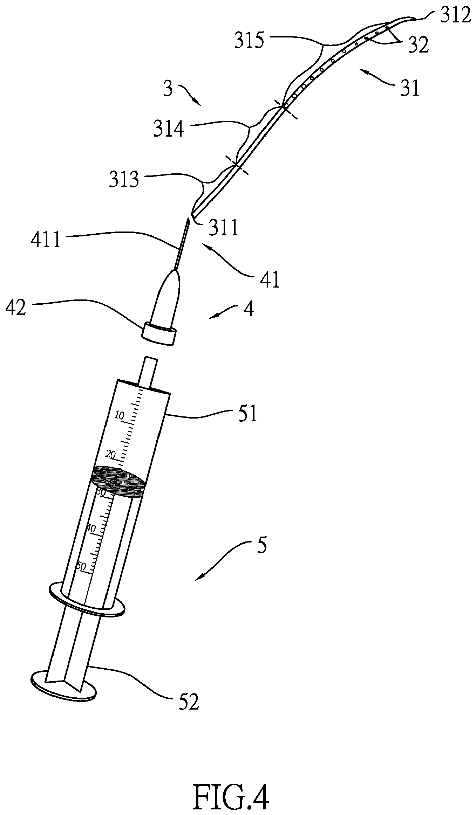

FIG. 4 is a perspective view of a nasal flushing device according to a first embodiment of the present invention.
FIG. 5 is a schematic drawing showing the nasal flushing device of the first embodiment being applied to the nasal cavity.
FIG. 6 is another schematic drawing showing the nasal flushing device of the first embodiment being applied to the nasal cavity.
FIG. 7 is a perspective view of the nasal flushing device according to a second embodiment of the present invention.

### DETAILED DESCRIPTION OF THE PREFERRED EMBODIMENT

Please refer to FIG. 3. FIG. 3 is a perspective view of a nasal flushing device according to a first embodiment of the present invention. The nasal flushing device comprises a catheter unit 3, a connecting unit 4, and an injection unit 5.

The catheter unit 3 has a catheter body 31 and a plurality of apertures 32 formed on the catheter body 31. The catheter body 31 has an open end 311 and a closed end 312 opposite the open end 311, and the closed end 312 curved such that the catheter body 31 is capable of entering deeply into the nasal meatus.

The catheter body 31 from the open end 311 to the closed end 312 is divided into a connecting section 313, a reaching section 314 and a flushing section 315. The connecting section 313 has a length from 1 to 5 cm, the reaching section 314 has a length from 1 to 5 cm, and the flushing section 315 has a length from 3 to 10cm. The plurality of apertures 32 on the catheter body 31 are disposed on the flushing section body 315, and wherein the apertures 32 further far away from the closed end 312 have larger inner diameters than the apertures 32 closer to the closed end 312. Furthermore, the catheter body 31 is made of silicon, latex or other soft and elastic substances, which can easily be navigated into the curved nasal meatus. Since average nasal meatus of patient with chronic sinusitis is not wider than 3 mm, a diameter of the catheter body 31 is not larger than 3mm.

The connecting unit 4 has a connecting portion 41 and an assembling portion 42. One end of the connecting portion 41 is provided with a connecting tube 411 connected to the open end 311, and another end of the connecting portion is connected to the assembling portion 42.

In addition, since the diameter of the connecting tube 411is smaller than the diameter of the catheter body 31 and it causes resistance to passing fluid, a length of the connecting tube 411should be as short as possible to reduce the resistance.

The injection unit 5 has a detachable syringe 51 and a putter set 52 provided in the syringe 51. The syringe 51 and the putter set 52 are used for compressing the fluid in the container, to form an injecting spout. There can be various combinations of the syringe 51 and the putter set 52 should not be limited by this embodiment.

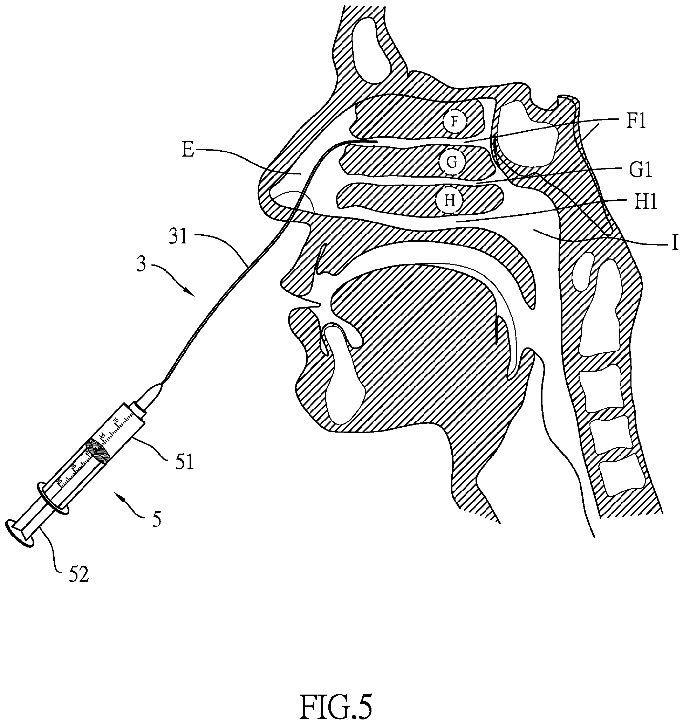

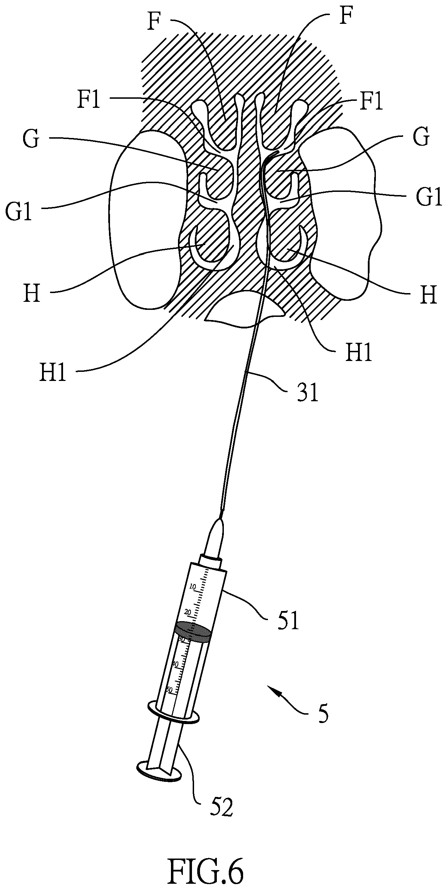

Please refer to FIG. 5 and FIG. 6 together. As showing the drawings, the nasal cavity has a nasal vestibule E, a superior turbinate F, a middle turbinate G, a inferior turbinate H, a upper nasal meatus F1, a middle nasal meatus G1 and a lower nasal meatus H1. The nasal vestibule usually is about 4 cm long, the upper nasal meatus F1, the middle nasal meatus G1 and the lower nasal meatus H1 are usually about 5 cm in length. Therefore, in the first embodiment, a length of the flushing section 315 of the catheter body 31 is 5cm, the plurality of apertures 32 are arranged apart about every 0.1cm to 0.2cm circumferentially. A total length of the catheter body 31 is about 17cm, the length of the flushing section 315 is 5cm, the length of the reaching section 314 in the nasal vestibule E is 4cm, the remaining length of the connecting section 313 is 8cm, which can be held by the user and provide further reaching length.

For actual application, the user inserts the flushing section 315 of the catheter body 31into the upper nasal meatus F1 or the middle nasal meatus G1, then pushes the putter set 52 to push the flushing fluid in the syringe 51, such that the flushing fluid spurs out from the apertures 32 on the flushing section 315 of the catheter body 31. Therefore, multiple thin spouts spur out with injecting angles nearly perpendicular to the catheter body 31. With injecting angles nearly perpendicular to the catheter body 31, the catheter body 31 is more likely free from counterforce movement during the injection, and the flushing fluid can quickly fill up the nasal meatuses.

Since the flushing section 315 is configured to be disposed into the superior nasal meatus F1 and the middle nasal meatus Gl, the flushing fluid can rinse the mucus or crust built up in this area directly.

Moreover, the catheter body 31 is made of silicone or latex material and has a small dimension, when the user insert the catheter body 31 into the nasal cavity, if he or she feels any discomfort, he or she can immediately stop to avoid damaging the tissue of the nasal cavity. Furthermore, if the catheter body 3 is not completely inserted into the upper nasal meatus F1, the spouts with injecting angles nearly perpendicular to the catheter body 31 injected from apertures 32 of the flushing section 315 are capable of rinsing most of the area. In addition, some spouts might reach up to a 30 cm distance, which can pass through some thin gaps into deeper areas for a better cleaning result. Even though, some spouts might reach up to a 30 cm distance, but the numerous number of spouts do not have high pressure to damage the mucosa.

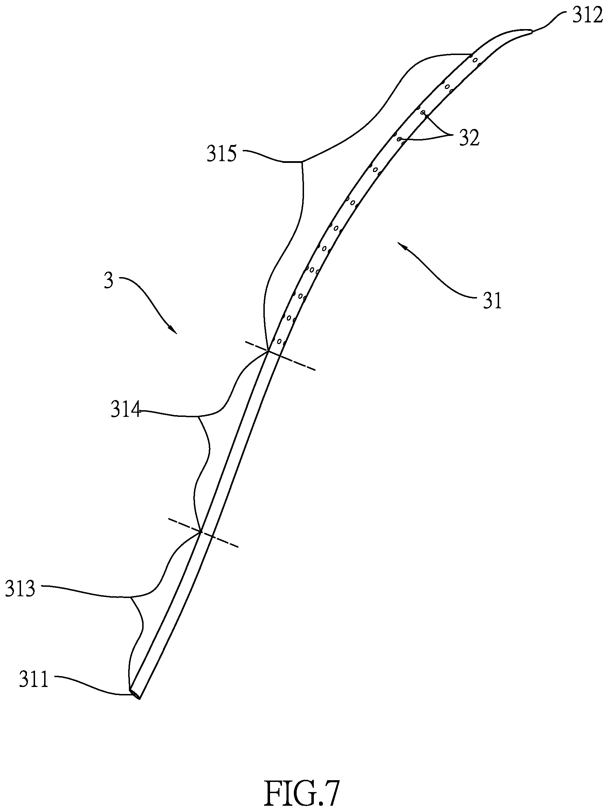

Please refer to FIG. 7. FIG. 7 is a perspective view of the nasal flushing device according to a second embodiment of the present invention. The second embodiment is substantially similar with the first embodiment, the difference is, every aperture 32 disposed on the flushing section 315 has an identical diameter, but the apertures 32 disposed further away from the closed end 312 on the flushing section 315 are arranged more closely together with respect to each other than the apertures disposed closer to the closed end 312.

Since the compressed spouts first release to lower pressure area, the spouts move towards to the closed end 312 and come out from the apertures. Because the apertures 32 disposed further away from the closed end are arranged more closely together with respect to each other than the apertures 32 disposed closer to the closed end 312, the entire flushing section 315 have relatively even flow and larger rinsing area.

Since the length of the flushing section 315 of the catheter body 31 is between 3 to10 cm, and the apertures 32 on the flushing section 315 further far away from the closed end 312 have larger inner diameters than the apertures 32 closer to the closed end 312 . The flushing fluid can rinse the upper nasal meatus F1 and the middle nasal meatus G1, and the plurality of apertures 32 with different diameter on the flushing section 315 can provide even pressure sprouts for more comfortable feeling to the user..

According to the above-mentioned description, the nasal flushing device utilizes the catheter unit 3 to deeply rinse the nasal cavity, and the combination of the catheter unit 3, the connecting unit 4, and the injection unit 5 provides portability to the user.

Although the present invention has been explained in relation to its preferred embodiment, it is to be understood that many other possible modifications and variations can be made without departing from the scope of the invention as hereinafter claimed.

## Claims

1. A device for nasal flushing treatment, comprising:
a catheter unit having a catheter body and a plurality of apertures formed on the catheter body, the catheter body having an open end; and
an injection unit having a detachable syringe and a putter set provided in the syringe connected to the open end, **characterized in that**:
the catheter body is made of a soft material, such as silicone or latex, and has a closed end opposite the open end, with a plurality of apertures near the closed end, and can be used by a user to insert into his/her nasal cavity, wherein multiple thin spouts can spur from the apertures to the nasal meatus.

2. The device for nasal flushing treatment as claimed in claim 1, further comprising a connecting unit, the connecting unit having a connecting portion and an assembling portion, one end of the connecting portion provided with a connecting tube connected to the open end, and another end of the connecting portion connected to the assembling portion.

3. The device for nasal flushing treatment as claimed in claim 2, wherein the catheter body from the open end to the closed end is divided into a connecting section, a reaching section and a flushing section, the connecting section having a length from 1 to 5 cm, the reaching section having a length from 1 to 5 cm, and the flushing section having a length from 3 to 10 cm, and wherein the closed end is slightly curved.

4. The device for nasal flushing treatment as claimed in claim 3, wherein the plurality of apertures on the catheter body is disposed on the flushing section, with the apertures located far away from the closed end having a larger diameter than the apertures being located closer to the closed end.

5. The device for nasal flushing treatment as claimed in claim 3 or 4, wherein the flushing section is configured to be disposed in a nasal meatus, the reaching section is configured to be disposed in a nasal vestibule, and the connecting section is configured to be exposed outside of a nostril, the apertures disposed further away from the closed end on the flushing section being arranged more closely together with respect to each other than the apertures disposed closer to the closed end.

## Patentansprüche

1. Nasenspülvorrichtung, umfassend:
eine Kathetereinheit, die einen Katheterkörper aufweist und eine Vielzahl an Öffnungen, die an dem Katheterkörper ausgebildet sind, wobei der Katheterkörper ein offenes Ende aufweist,
und
eine Injektionseinheit, die eine abnehmbare Spritze und einen Ladesatz aufweist, der in der Spritze bereitgestellt ist, die mit dem offenen Ende verbunden ist, **dadurch gekennzeichnet, dass**
der Katheterkörper aus einem weichen Material hergestellt ist, wie zum Beispiel Silikon oder Latex, und ein geschlossenes Ende entgegengesetzt zu dem offenen Ende aufweist, mit einer Mehrzahl an Öffnungen nahe dem geschlossenen Ende, und von einem Benutzer verwendet werden kann, um in seine/ihre Nasenhöhle geschoben zu werden, wobei mehrere dünne Ausgüsse von den Öffnungen zu dem Nasenkanal führen können.

2. Nasenspülvorrichtung gemäß Anspruch 1, die ferner eine Verbindungseinheit umfassend, wobei die Verbindungseinheit einen Verbindungsabschnitt und einen Anordnungsabschnitt aufweist, wobei ein Ende des Verbindungsabschnitts mit einem Verbindungsrohr ausgestattet ist, das mit dem offenen Ende verbunden ist und wobei ein anderes Ende des Verbindungsabschnitts mit dem Aufbauabschnitt verbunden ist.

3. Nasenspülvorrichtung gemäß Anspruch 2, bei der der Katheterkörper von dem offenen Ende zu dem geschlossenen Ende in einen Verbindungsbereich, einen Erstreckungsbereich und einen Spülbereich aufgeteilt ist, wobei der Verbindungsbereich eine Länge von 1 bis 5 cm hat, der Erstreckungsbereich eine Länge von 1 bis 5 cm hat und der Spülbereich eine Länge von 3 bis 10 cm hat, und wobei das geschlossene Ende leicht gekrümmt ist.

4. Nasenspülvorrichtung gemäß Anspruch 3, bei der die Vielzahl an Öffnungen auf dem Katheterkörper an dem Spülbereich angebracht ist, wobei die Öffnungen, die von dem geschlossenen Ende weit weg angeordnet sind, einen größeren Durchmesser aufweisen als die Öffnungen, die näher an dem geschlossenen Ende angeordnet sind.

5. Nasenspülvorrichtung gemäß Anspruch 3 oder 4, bei der der Spülbereich dazu konfiguriert ist, in einen Nasenkanal geführt zu werden, wobei der Erstreckungsbereich dazu konfiguriert ist, in einen Naseneingang geführt zu werden, und wobei der Verbindungsbereich dazu konfiguriert ist, außerhalb eines Nasenlochs exponiert zu werden, wobei die Öffnungen, die weiter weg von dem geschlossenen Ende an dem Spülbereich angeordnet sind, im Verhältnis zueinander näher aneinander angeordnet sind als die Öffnungen, die näher an dem geschlossenen Ende angeordnet sind.

## Revendications

1. Dispositif de traitement de lavage nasal, comprenant:
une unité de cathéter comportant un corps de cathéter et une pluralité d'ouvertures formées sur le corps de cathéter, le corps de cathéter ayant une extrémité ouverte; et
une unité d'injection ayant une seringue détachable et un jeu de placement ménagé dans la seringue raccordée à l'extrémité ouverte, **caractérisé en ce que**:
le corps de cathéter est constitué d'une matière souple, telle que le silicone ou le latex, et a une extrémité fermée opposée à l'extrémité ouverte, avec une pluralité d'ouvertures près de l'extrémité fermée, et peut être utilisé par un utilisateur pour insertion dans sa cavité nasale, dans lequel de multiples jets fins peuvent jaillir des ouvertures vers le méat nasal.

2. Dispositif de traitement de lavage nasal selon la revendication 1, comprenant en outre une unité de raccordement, l'unité de raccordement ayant une portion de raccordement et une portion d'assemblage, une extrémité de la portion de raccordement étant pourvue d'un tube de raccordement raccordé à l'extrémité ouverte, et une autre extrémité de la portion de raccordement raccordée à la portion d'assemblage.

3. Dispositif de traitement de lavage nasal selon la revendication 2, dans lequel le corps de cathéter de l'extrémité ouverte à l'extrémité fermée est divisé en une section de raccordement, une section de portée et une section de lavage, la section de raccordement ayant une longueur de 1 à 5 cm, la section de portée ayant une longueur de 1 à 5 cm, et la section de lavage ayant une longueur de 3 à 10 cm, et dans lequel l'extrémité fermée est légèrement incurvée.

4. Dispositif de traitement de lavage nasal selon la revendication 3, dans lequel la pluralité d'ouvertures sur le corps de cathéter est disposée sur la section de lavage, avec les ouvertures situées à distance de l'extrémité fermée ayant un plus grand diamètre que les ouvertures situées plus près de l'extrémité fermée.

5. Dispositif de traitement de lavage nasal selon la revendication 3 ou 4, dans lequel la section de lavage est configurée pour être disposée dans un méat nasal, la section de portée est configurée pour être disposée dans un vestibule nasal, et la section de raccordement est configurée pour être exposée à l'extérieur d'une narine, les ouvertures disposées plus à distance de l'extrémité fermée sur la section de lavage étant agencées plus étroitement conjointement les unes avec les autres que les ouvertures disposées plus près de l'extrémité fermée.
